(19) Europäisches Patentamt
European Patent Office
Office européen des brevets

(11) Veröffentlichungsnummer: **0 093 852**
**A1**

(12) # EUROPÄISCHE PATENTANMELDUNG

(21) Anmeldenummer: **83102405.4**

(22) Anmeldetag: **11.03.83**

(51) Int. Cl.³: **C 07 D 321/00**, C 11 B 9/00
// A61K7/46

(30) Priorität: **30.04.82 DE 3216084**

(43) Veröffentlichungstag der Anmeldung: **16.11.83**
**Patentblatt 83/46**

(84) Benannte Vertragsstaaten: **AT CH DE FR GB LI NL**

(71) Anmelder: **CHEMISCHE WERKE HÜLS AG, - RSP Patente / PB 15 - Postfach 13 20, D-4370 Marl 1 (DE)**

(72) Erfinder: **Burzin, Klaus, Dr., Wellerfeldweg 164, D-4370 Marl (DE)**

(54) **Makrocyclische Dicarbonsäureester, Verfahren zu ihrer Herstellung und ihre Verwendung als Riechstoffe.**

(57) Gegenstand der Erfindung sind makrocyclische Dicarbonsäureester der allgemeinen Formel 1,

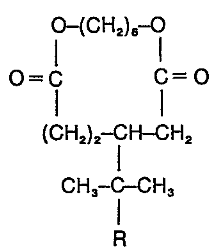

1

in der R = H oder ein Alkylrest mit 1 bis 5 Kohlenstoffatomen ist, ihre Verwendung als Riechstoffe sowie ein Verfahren zu ihrer Herstellung.



EP 0 093 852 A1

Makrocyclische Dicarbonsäureester, Verfahren zu ihrer Herstellung und ihre Verwendung als Riechstoffe

Gegenstand der Erfindung sind makrocyclische Dicarbonsäureester der allgemeinen Formel 1,

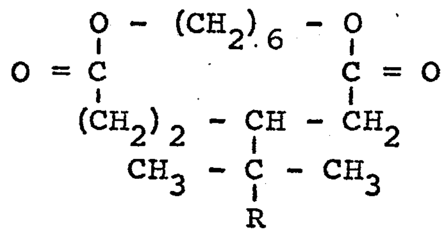

1

in der R = H oder ein Alkylrest mit 1 bis 5 Kohlenstoffatomen ist, ihre Verwendung als Riechstoffe sowie ein Verfahren zu ihrer Herstellung.

Schon seit einiger Zeit besteht ein Bedürfnis nach Riechstoffen mit Holzcharakter, die eine lange Haftdauer aufweisen. Unter den makrocyclischen Verbindungen mit holzigem Geruch haben Cyclododecylderivate der Formel 2 eine besondere Bedeutung erlangt.

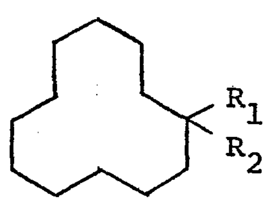

2

$R_1$ = H, $R_2$ = $CH_2$-O-AlK (DE-PS 29 28 347)
$R_1$ = H, $R_2$ = O-AlK (CH-PS 443 536)
$R_1$ = $CH_3$, $R_2$ = O-AlK (DE-B2 21 52 016)

Diese Riechstoffe mit Holzcharakter weisen bei Raumtemperatur eine für spezielle Einsatzzwecke - wie z. B. die Parfümierung von Holzimitationen aus Kunststoffen - nicht ausreichende Haftfestigkeit auf.

Es wurde jetzt überraschend gefunden, daß die neuen makrocyclischen Dicarbonsäureester der allgemeinen Formel 1 sowohl einen intensiven Holzgeruch als auch eine große Haftfestigkeit aufweisen und vorteilhaft dort verwendet werden können, wo eine besonders lange Haftdauer gefordert wird.

Grundsätzlich sind makrocyclische Dicarbonsäureester als Riechstoffe bekannt. Besondere Bedeutung haben cyclische Ester aus Brassylsäure bzw. Dodecandisäure und Ethylenglykol erlangt (DE-B2 25 47 267, DE-A 19 29 550). Auch cyclische Ester aus methylsubstituierten Dicarbonsäuren und Ethylenglykol mit Ringgliederzahlen von 16 bis 19 wurden bereits beschrieben (DE-OS 29 29 864). Alle bisher bekannten makrocyclischen Dicarbonsäureester weisen jedoch moschusähnliche Gerüche unterschiedlicher Intensität auf. Es ist daher überraschend, daß die erfindungsgemäßen makrocyclischen Dicarbonsäureester Riechstoffe mit starkem Holzcharakter darstellen.

Die Erfindung betrifft ferner ein Verfahren zur Herstellung der makrocyclischen Dicarbonsäureester nach Formel 1. Dies ist dadurch gekennzeichnet, daß man eine Verbindung der allgemeinen Formel 3,

$$\begin{array}{c}
 \qquad\qquad\qquad\qquad\qquad\quad R \\
 \qquad\qquad\qquad\qquad\qquad\quad | \\
 \qquad O \qquad\qquad CH_3 - C - CH_3 \qquad O \\
 \qquad \| \qquad\qquad\qquad\qquad | \qquad\qquad\qquad \| \\
R' - O - C - (CH_2)_2 - CH - CH_2 - C - O - R'
\end{array} \qquad \underline{3}$$

in der R = H oder ein Alkylrest mit 1 bis 5 Kohlenstoffatomen ist und R' für ein H-Atom oder einen $C_1$-$C_4$-Alkylrest steht, mit Hexandiol-(1,6) zu einem linearen Polyester umsetzt und den Polyester anschließend unter vermindertem Druck bei Temperaturen von 200 bis 300 °C thermolysiert.

Die Herstellung der als Ausgangsverbindungen eingesetzten linearen Polyester aus Hexandiol-(1,6) und Verbindungen der allgemeinen Formel 3 kann nach bekannten Methoden, wie sie z. B. von Korshak und Vinogradova in "Polyesters", Seite 153 ff. (Pergamon Press 1965), beschrieben sind, erfolgen. Vorzugsweise werden die Polyester durch Veresterung der jeweiligen Dicarbonsäuren mit Hexandiol-(1,6) ohne Katalysator und ohne Schleppmittel bei Temperaturen zwischen 150 bis 220 °C hergestellt.

Bei der anschließenden Thermolyse hat es sich bewährt, Polyester einzusetzen, deren Säurezahl unter 10 mg KOH/g liegt, um durch Carboxylendgruppen induzierte Nebenreaktionen zu vermeiden. Der mittlere Polymerisationsgrad sollte zwischen 3 und 200, vorzugsweise zwischen 10 und 50, liegen.

Die Thermolysetemperaturen liegen zwischen 200 bis 300 °C. Unterhalb 200 °C verläuft die Thermolyse zu langsam, oberhalb 300 °C treten unerwünschte Nebenreaktionen auf. Als bevorzugter Bereich hat sich der Bereich zwischen 250 und 280 °C erwiesen. In diesem Bereich erfolgt eine zügige Bildung des cyclischen Dicarbonsäureesters, ohne daß es zu nennenswerten Nebenreaktionen kommt. Die Thermolysereaktion wird unter vermindertem Druck durchgeführt. Besonders glatt verläuft die Reaktion bei Drücken unter 1 mbar.

Die Thermolyse wird in Gegenwart üblicher Depolymerisierungskatalysatoren (Magnesium-, Zinn-, Bleisalze bzw. -oxide) vorgenommen. Zur Verbesserung der Rührbarkeit empfiehlt es sich, der Schmelze O,O-Dialkyl-(3,5-di-tert.-butyl-4-hydroxy-benzyl)-phosphonat (vgl. DE-B2 25 47 267) zuzusetzen.

Die Verbindungen der allgemeinen Formel 3 können nach bekannten Verfahren durch Alkylierung von Phenol, Reduktion zu 4-alkylsubstituierten Cyclohexanolen und anschließende Oxidation zu den entsprechenden Dicarbonsäuren hergestellt werden (vgl. N. K. Taikova, A. E. Kulikova und E. N. Zilberman, Zh. Prikl. Khim. 39 (3), 668 bis 671 (1966).

Die erfindungsgemäßen Verbindungen weisen gegenüber den bekannten makrocyclischen Dicarbonsäureestern überraschenderweise den Vorteil auf, daß sie neben einer außerordentlich großen Haftdauer einen starken Holzcharakter besitzen. Gegenüber den bekannten Cyclododecylderivaten mit Holzcharakter weisen die erfindungsgemäßen Verbindungen eine deutlich längere Haftdauer auf. Während das Haftvermögen von Cyclododecylderivaten der Formel 2 auf Riechstäbchen im Normklima etwa 1 bis 2 Wochen beträgt, haftet der Geruch der makrocyclischen Dicarbonsäureester 1 unter den gleichen Bedingungen länger als 2 Monate.

Die erfindungsgemäßen Verbindungen werden in Mischungen mit anderen Riechstoffen in Parfümierungsmitteln, z. B. in Mengen von 0,01 bis 50 Gewichtsprozent, vorzugsweise 1 bis 25 Gewichtsprozent, bezogen auf das Gesamtgewicht der Riechstoffkomposition, eingesetzt.

Wegen der starken Holznote und der überdurchschnittlichen Haftdauer ist das Anwendungsgebiet der erfindungsgemäßen Verbindungen sehr groß. Die Verbindungen finden sowohl Verwendung zur Parfümierung von Tabak und Holzimitationen aus Kunststoff, als auch in Parfümkompositionen, z. B. für Kosmetika, Extraits, Deo-Sprays, Seifen und andere Waschmittel.

## Beispiel 1

Herstellung von 11-Isopropyl-1,8-dioxa-cyclotetradecan-9,14-dion

100 g eines Polyesters aus 3-Isopropyl-adipinsäure und Hexandiol-(1,6) mit einem Polymerisationsgrad von 19 und einer Säurezahl von 5 mg KOH/g werden in einem Destillationskolben einer Vakuumdestillationsapparatur mit 2 g Zinn-II-acetat innig vermischt. Danach wird die Mischung bei einem Vakuum von weniger als 1 mbar auf 270 °C erhitzt, wobei unter Aufschäumen des Polyesters das Reaktionsprodukt überdestilliert. Die Thermolyse ist nach 4 bis 6 Stunden beendet. Man erhält 83 g Rohprodukt und nach einer Destillation über eine 10 cm-Vigreux-Kolonne 78 g 11-Isopropyl-1,8-dioxa-cyclotetradecan-9,14-dion mit einem Siedepunkt von 118 °C/0,05 mbar, entsprechend einer Ausbeute von 78 %, bezogen auf eingesetzten Polyester.

| | |
|---|---|
| Geruchsberuteilung: | holzig-herb, sehr ausgiebig |
| Haftdauer: | länger als 2 Monate |

## Beispiel 2

Herstellung von 11-tert.-Butyl-1,8-dioxa-cyclotetradecan-9,14-dion

100 g eines Polyesters aus 3-tert.-Butyl-adipinsäure und Hexandiol-(1,6) mit einem Polymerisationsgrad von 24 und einer Säurezahl von 6 mg KOH/g werden in einem Destillationskolben einer Vakuumdestillationsapparatur mit 2,5 g Di-n-octyl-zinnoxid intensiv vermischt. Das Reaktionsgemisch wird bei einem Vakuum von 0,2 mbar und einer Temperatur von 268 °C 5 Stunden thermolysiert.

Man erhält 79 g Rohprodukt und nach einer Destillation über eine 10 cm-Vigreux-Kolonne 73,5 g 11-tert.-Butyl-1,8-dioxa-cyclotetradecan-9,14-dion mit einem Siedepunkt von 127 °C/0,05 mbar, entsprechend einer Ausbeute von 73,5 %, bezogen auf eingesetzten Polyester.

Geruchsbeurteilung: holzig, ausgiebig
Haftdauer: länger als 2 Monate

Beispiel 3

Herstellung von 11-(1,1,3-Trimethylbutyl)-1,8-dioxacyclotetradecan-9,14-dion

Analog Beispiel 2 werden 200 g Polyester aus 3-(1,1,3-Trimethylbutyl)-adipinsäure und Hexandiol-(1,6) mit einem Polymerisationsgrad von 18, Säurezahl 4 mg KOH/g, bei 270 °C und einem Vakuum von 0,1 mbar 4 Stunden thermolysiert.

Man erhält 164 g Rohprodukt und nach einer Destillation über eine 10 cm-Vigreux-Kolonne 152 g 11-(1,1,3-Trimethylbutyl)-1,8-dioxacyclotetradecan-9,14-dion mit einem Siedepunkt von 149 °C/0,02 mbar, entsprechend einer Ausbeute von 76 %, bezogen auf eingesetzten Polyester.

Geruchsbeurteilung: holzartig, blumig
Haftdauer: etwa 3 Monate

Beispiel 4

Herstellung von 11-(1,1,3,3-Tetramethylbutyl)-1,8-dioxacyclotetradecan-9,14-dion

100 g eines Polyesters aus 3-(1,1,3,3-Tetramethylbutyl)-adipinsäure und Hexandiol-(1,6) mit einem mittleren Polymerisationsgrad von 22 und einer Säurezahl von 7 mg KOH/g, 2,5 g Dioctylzinnoxid und 2,5 g O,O-Di-Octadecyl-(3,5-Di-tert.-butyl-4-hydroxy-benzyl)-phosphonat werden in einer üblichen Vakuumdestillationsapparatur intensiv vermischt und bei einem Vakuum von 0,1 mbar auf 270 °C 6 Stunden erhitzt.

Man erhält 79,5 g Rohprodukt und nach einer Destillation über eine 10 cm-Vigreux-Kolonne 72 g 11-(1,1,3,3-Tetramethylbutyl)-1,8-dioxa-cyclotetradecan-9,14-dion mit einem Siedepunkt von 147 °C/0,01 mbar, entsprechend einer Ausbeute von 72 %, bezogen auf eingesetzten Polyester.

Geruchsbeurteilung: holzartig, blumig
Haftdauer: etwa 3 Monate

Beispiel 5

Eine Riechstoffkomposition mit Holznote wird beispielsweise durch Mischen folgender Komponenten hergestellt:

| | Gewichtsteile |
|---|---|
| Cumarin | 20 |
| Heliotropin | 30 |
| Phenylethylalkohol | 100 |
| Geraniol | 50 |
| Rhodinol | 100 |
| Vetiveröl (Bourbon) | 30 |
| Perubalsam | 70 |
| Patchouliöl | 30 |
| Cedrylacetat | 100 |
| Sandelholz, ostindisch | 40 |
| Eichenmoos, jugoslawisch | 20 |
| α-Amylzimtaldehyd | 50 |
| Rose de may (10 %ig in Phthalsäurediethylester) | 40 |

| | Gewichtsteile |
|---|---|
| Linalylacetat | 30 |
| Bergamottöl Reggio | 80 |
| Phthalsäurediethylester | 150 |
| | 940 |

Durch Zusatz von 60 Gewichtsteilen 11-tert.-Butyl-1,8-dioxa-cyclotetradecan-9,14-dion erhält obige Komposition eine starke Holznote und eine Erhöhung der Haftfestigkeit. Ähnliche Ergebnisse werden durch Zusatz von 30 Gewichtsteilen 11-(1,1,3,3-Tetramethylbutyl)-1,8-dioxa-cyclotetradecan-9,14-dion bzw. 50 Gewichtsteilen 11-(1,1,3-Trimethylbutyl)-1,8-dioxa-cyclotetradecan-9,14-dion erreicht.

Patentansprüche:

1. Makrocyclische Dicarbonsäureester der allgemeinen Formel

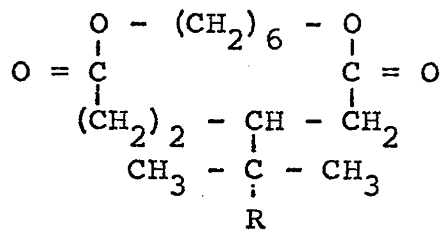

,

in der R = H oder ein Alkylrest mit 1 bis 5 Kohlenstoffatomen ist.

2. 11-Isopropyl-1,8-dioxacyclotetradecan-9,14-dion.

3. 11-tert.-Butyl-1,8-dioxacyclotetradecan-9,14-dion.

4. 11-(1,1,3-Trimethylbutyl)-1,8-dioxacyclotetradecan-9,14-dion.

5. 11-(1,1,3,3-Tetramethylbutyl)-1,8-dioxacyclotetradecan-9,14-dion.

6. Verfahren zur Herstellung der makrocyclischen Diester gemäß Anspruch 1,
dadurch gekennzeichnet,
daß man eine Verbindung der allgemeinen Formel

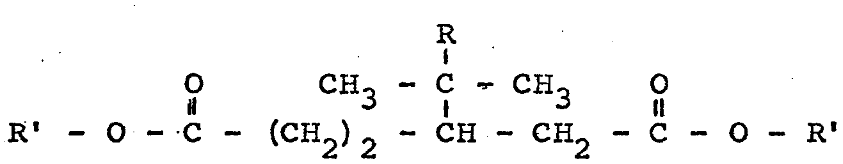

,

in der R = H oder ein Alkylrest mit 1 bis 5 Kohlenstoffatomen ist und R' für ein H-Atom oder einen $C_1$-$C_4$-Alkylrest steht, mit Hexandiol-(1,6) zu einem

linearen Polyester umsetzt und den Polyester anschließend unter vermindertem Druck bei Temperaturen von 200 bis 300 °C thermolysiert.

7. Verwendung der Verbindungen nach Anspruch 1 zur Herstellung von Riechstoffkompositionen.

Europäisches Patentamt

# EUROPÄISCHER RECHERCHENBERICHT

0093852 Nummer der Anmeldung

EP 83 10 2405

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 023 612 (HAARMANN & REIMER)<br>* Ansprüche 1, 3 *<br>--- | 1,7 | C 07 D 321/00<br>C 11 B 9/00 //<br>A 61 K 7/46 |
| A | Patent Abstracts of Japan Band 5, Nr. 111, 18. Juli 1981 & JP-A-56-51474<br>--- | | |
| A | EP-A-0 022 460 (CHEMISCHE WERKE HÜLS AG)<br>& DE-A-2 928 347 (Kat. D)<br>----- | | |
| | | | RECHERCHIERTE SACHGEBIETE (Int. Cl. 3) |
| | | | A 61 K 7/46<br>C 07 D 321/00<br>C 11 B 9/00 |

Der vorliegende Recherchenbericht wurde für alle Patentansprüche erstellt.

| Recherchenort | Abschlußdatum der Recherche | Prüfer |
|---|---|---|
| BERLIN | 23-06-1983 | PHILLIPS N.G.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82